# EUROPEAN PATENT SPECIFICATION

(11) **EP 3 503 969 B1**
(45) Date of publication and mention of the grant of the patent: **23.12.2020**
(21) Application number: 17761682.8
(22) Date of filing: 22.08.2017
(51) Int. Cl.: A61N 1/375, A61N 1/05, A61N 1/36

(54) **LEADLESS DEVICE FOR NEUROMODULATION OF THE ADRENAL GLAND**
LEITUNGSLOSES GERÄT ZUR NEUROMODULATION DER NEBENNIERE
DISPOSITIF SANS FIL POUR LA NEUROMODULATION DE LA GLANDE SURRÉNALE

(30) Priority: 23.08.2016 US 201662378503 P
(43) Date of publication of application: 03.07.2019
(73) Proprietor: Boston Scientific Neuromodulation Corporation, Valencia, CA 91355 (US)
(72) Inventor: SWANSON, Lynne E., Edina, Minnesota 55424 (US); CLARK, Bryan A., Forest Lake, Minnesota 55025 (US); WERNER, Dennis B., Big Lake, Minnesota 55309 (US); STOFFREGEN, William C., Lake Elmo, Minnesota 55042 (US); GOVEA, Michael X., Castaic, California 91384 (US); BRILL, Natalie A., Valencia, California 91355 (US)
(74) Representative: Vossius & Partner Patentanwälte Rechtsanwälte mbB
(86) International application number: PCT/US2017/048088
(87) International publication number: WO 2018/039287

(56) References cited:
- US-A1- 2010 185 249
- US-A1- 2010 305 664
- US-A1- 2015 148 878
- US-A1- 2016 038 749

## Description

### TECHNICAL FIELD

The present disclosure relates to medical devices for providing stimulation therapy. More specifically, the disclosure relates to devices for delivering leadless stimulation therapy to an adrenal gland of a patient.

### BACKGROUND

Electrical stimulation may be therapeutic in a variety of diseases and disorders. Stimulation may be applied by electrical leads that extend from a pulse generator to a target site. In other instances, stimulation may be applied by a leadless implantable medical device (IMD). Such devices have been used, for example, for cardiac pacing/stimulation.

Dysfunction of the adrenomedullary system, or abnormal levels of catecholamines, such as norepinephrine, epinephrine and dopamine, have been associated with symptoms including depression, fibromyalgia, chronic fatigue, chronic pain, migraines, orthostatic intolerance, Postural Orthostatic Tachycardia Syndrome (POTS), and other disease states. Electrical current, or other modes of energy, may be delivered to the adrenal gland to cause an up-regulation or down-regulation of catecholamine release into the bloodstream

Preganglionic sympathetic axons of the splanchnic nerve terminate at chromaffin cells in the medulla of the adrenal glands. Two populations of chromaffin cells, one releasing epinephrine and one norepinephrine, demonstrate different innervation patterns. As a result, different stimulation patterns may be used to alter these levels independently to drive the abnormal levels of catecholamines toward normal levels thereby providing therapy and/or treatment for one or more of the various disease states associated therewith.

US 2010/185249 A1 describes a method of treating a patient. The method comprises: implanting a stimulation lead comprising an electrode near an adrenal gland of the patient; implanting a neurostimulator within the patient; and applying electrical current from the electrode to the adrenal gland to treat a pulmonary condition of the patient.

US 2010/305664 A1 describes a method of treating a disorder. The method comprises: placing a stimulation lead on neural tissue in communication with an adrenal gland; and delivering an electrical waveform from a neurostimulator to the stimulation lead to release catecholamines from the adrenal gland to treat hypoglycemia.

US 2016/038749 A1 describes systems and methods for coordinating treatment of abnormal heart activity using multiple implanted devices within a patient.

### SUMMARY

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

While multiple embodiments are disclosed, still other embodiments of the present disclosure will become apparent to those skilled in the art from the following detailed description, which shows and describes illustrative embodiments of the disclosure. Accordingly, the drawings and detailed description are to be regarded as illustrative in nature and not restrictive.

### BRIEF DESCRIPTION OF THE DRAWINGS

FIG. 1 is an illustration of an example implantable medical device arranged with an adrenal gland of a patient in accordance with embodiments of the disclosure.
FIG. 2 is a schematic block diagram of an implantable system having an IMD and a control device in accordance with embodiments of the disclosure.
FIG. 3 is a schematic block diagram of an IMD in accordance with embodiments of the disclosure.
FIG. 4A is an illustration of an example leadless implantable medical device in accordance with embodiments of the disclosure.
FIG. 4B is an illustration of the example leadless implantable medical device, shown in FIG. 4A, attached to a patient's adrenal gland in accordance with embodiments of the disclosure.
FIG. 5A is an illustration of an example leadless implantable medical device and a deployment system in accordance with embodiments of the disclosure.
FIG. 5B is a cross-sectional illustration of the example leadless implantable medical device and the deployment system shown in FIG. 5A in accordance with embodiments of the disclosure.
FIG. 6 is an illustration of an example leadless implantable medical device having a mechanical attachment structure in accordance with embodiments of the disclosure.
FIG. 7 is an illustration of an example leadless implantable medical device having another example mechanical attachment structure in accordance with embodiments of the disclosure.
FIG. 8 is an illustration of an example leadless implantable medical device having another example mechanical attachment structure in accordance with embodiments of the disclosure.
FIG. 9A is an illustration of an example leadless implantable medical device including a leadless body portion having flexible portions in accordance with embodiments of the disclosure.
FIG. 9B is an illustration of the example leadless implantable medical device, shown in FIG. 9A, arranged with a portion of a patient's adrenal gland in accordance with embodiments of the disclosure.
FIG. 10 is an illustration of another example leadless implantable medical device in accordance with embodiments of the disclosure.

While the disclosure is amenable to various modifications and alternative forms, specific embodiments have been shown by way of example in the drawings and are described in detail below. The intention, however, is not to limit the disclosure to the particular embodiments described. On the contrary, the disclosure is intended to cover all modifications, equivalents, and alternatives falling within the scope of the disclosure as defined by the appended claims.

As the terms are used herein with respect to ranges of measurements (such as those disclosed immediately above), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error, differences in measurement and/or manufacturing equipment calibration, human error in reading and/or setting measurements, adjustments made to optimize performance and/or structural parameters in view of differences in measurements associated with other components, particular implementation scenarios, imprecise adjustment and/or manipulation of objects by a person or machine, and/or the like.

Although the term "block" may be used herein to connote different elements illustratively employed, the term should not be interpreted as implying any requirement of, or particular order among or between, various blocks disclosed herein. Similarly, although illustrative methods may be represented by one or more drawings (e.g., flow diagrams, communication flows, etc.), the drawings should not be interpreted as implying any requirement of, or particular order among or between, various steps disclosed herein. However, certain embodiments may require certain steps and/or certain orders between certain steps, as may be explicitly described herein and/or as may be understood from the nature of the steps themselves (e.g., the performance of some steps may depend on the outcome of a previous step). Additionally, a "set," "subset," or "group" of items (e.g., inputs, algorithms, data values, etc.) may include one or more items, and, similarly, a subset or subgroup of items may include one or more items. A "plurality" means more than one.

### DETAILED DESCRIPTION

Various aspects of the present disclosure relate to apparatuses and systems directed toward neuroendocrine modulation of a patient's adrenal gland. Stimulation for depolarization of chromaffin cell membrane or splanchnic nerve effect within the adrenal medulla, for pain and non-pain symptoms of chronic fatigue syndrome and fibromyalgia and other disease states with neurotransmitter or neurohormonal dysfunction. Chromaffin cells in the adrenal medulla synthesize, store, and secret catecholamines (*e.g.,* norepinephrine, epinephrine). Adrenal gland stimulation may lead to an increase, or produce a blocking effect in the activity of several of the catecholamine biosynthetic enzymes, and to an increase or decrease in the rate of catecholamine biosynthesis or chromaffin cell exocytosis thereby causing secretion or blocking of catecholamines and of other chromaffin granule constituents from the chromaffin cells.

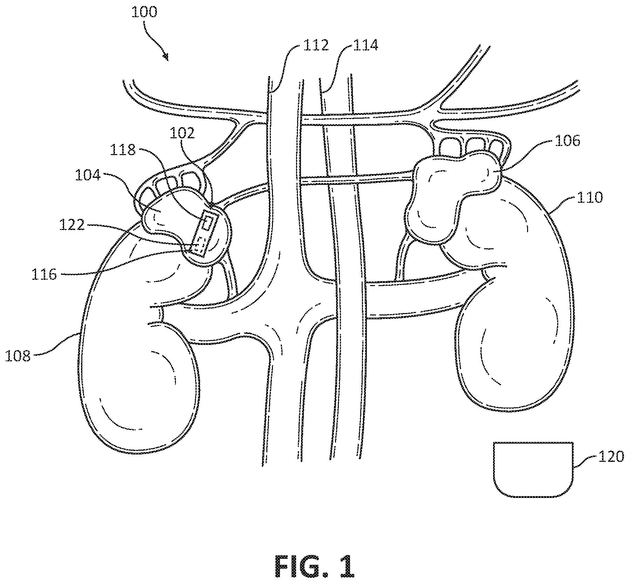

FIG. 1 is a schematic illustration of an implantable system including an implantable medical device (IMD) 102 attached to a portion of a patient's adrenal gland 104 in accordance with embodiments of the present disclosure. The anatomy 100 shown in FIG. 1 includes adrenal glands 104, 106 (with Gerota's fascia), which are located above the patient's kidneys 108, 110, which are located on either side of the patient's vena cava 112 and aorta 114. The IMD 102 may include a leadless body portion 116 configured to engage the adrenal gland 104 (or periadrenal connective tissue), and at least one electrode 118 arranged with the leadless body portion. The electrode 118 may be configured to deliver stimulation energy to modulate catecholamine release from chromaffin cells within the adrenal gland 104. More specifically, the IMD 102 may be configured to target a therapy location that includes a portion of an adrenal gland that is identified as being likely to be associated with an adrenal gland condition. For example, in implementations used for treating disorders such as chronic fatigue syndrome, fibromyalgia, orthostatic intolerance, irritable bowel syndrome (IBS), Crohn's disease, mood disorders/depression, or other disease states with neurotransmitter or neurohormonal dysfunction, a clinician may insert the IMD 102 near or on a region of one or both of the patient's adrenal glands 104, 106 associated with the disorder. The IMD 102 may deliver stimulation energy to a selected region via the electrode 118.

As shown in FIG. 1, the IMD 102 is a wireless electrode stimulator assembly. The system 100 may also include a controller 120 that is configured to communicate with the IMD 102. In certain instances, the controller 120 may be co-implanted and may provide therapy and/or diagnostic data about the patient and/or the controller 120. In other instances, the controller 120 may be arranged external to the patient. The IMD 102 may include circuitry to sense and analyze the adrenal gland 104 electrical activity, and to determine if and when a pacing electrical pulse needs to be delivered and, in instances having multiple IMDs 102 (configured as described above), by which of the IMDs 102 the pulse should be delivered. In embodiments having multiple IMDs 102, an IMD 102 may be attached to a portion of the patient's other adrenal gland 106. The sensing capability may be made possible by having sense electrodes included within the physical assembly of the controller 120. In certain instances, the IMD 102 may include sensing capability, and may communicate sensed information to other IMDs 102 and/or to the controller 120. In such instances, the IMD 102 may include an additional electrode(s) 122. Either of the electrode(s) 118 and/or the additional electrode 122 may be configured to provide stimulation energy or sense information about the adrenal gland 104. In certain instances, the IMD 102 may include more than one additional electrode 122. In certain instances, the controller 120 may transmit charge energy and data, such as stimulation energy trigger signals, pacing amplitude information, and pulse width information to the IMD 102 via radio-frequency (RF) communications, acoustic communications, inductive communications, electrical communications, microwave communications, conductive communications, and/or the like.

In certain instances, implantable systems such the IMD 102 may be unipolar, multipolar (e.g., bipolar, quad-polar, etc.) or configurable such that a unipolar or multipolar operation can be selected. In a unipolar system, one of the electrode 118 and the electrode 122 of the IMD 102 acts as one pole of an electrical system, and the second pole of the electrical system may be located remotely from the electrode. For example, the second pole of the electrical system may be located on the housing of the IMD 102 or on a wire connected to the IMD 102. Various other configurations for unipolar devices are known in the art.

In a bipolar system, the IMD 102 may use the electrode 118 and the electrode 122. For example, the electrode 118 and the electrode 122 may be disposed on the body of the IMD 102 as a tip electrode and a ring electrode). The electrode 118 and the electrode 122 may act as the two electrical poles of the IMD 102. Various other configurations for bipolar electrodes are known in the art. The electrode 118 and the electrode 122 also may be configured to sense certain physiological attributes of the adrenal gland 104. For example, the catecholamine levels in the patient can be received by one of the electrode 118 and the electrode 122 and transmitted to a remote location (e.g., the controller 120). In addition, other sensing mechanisms that are known in the art may be placed within, on or near the IMDs 102, and may include, for example, physiological sensors, pressure sensors, motion sensors, activity sensors, posture sensors, and/or the like, which may communicate directly with the IMD 102 and/or directly with the controller 120.

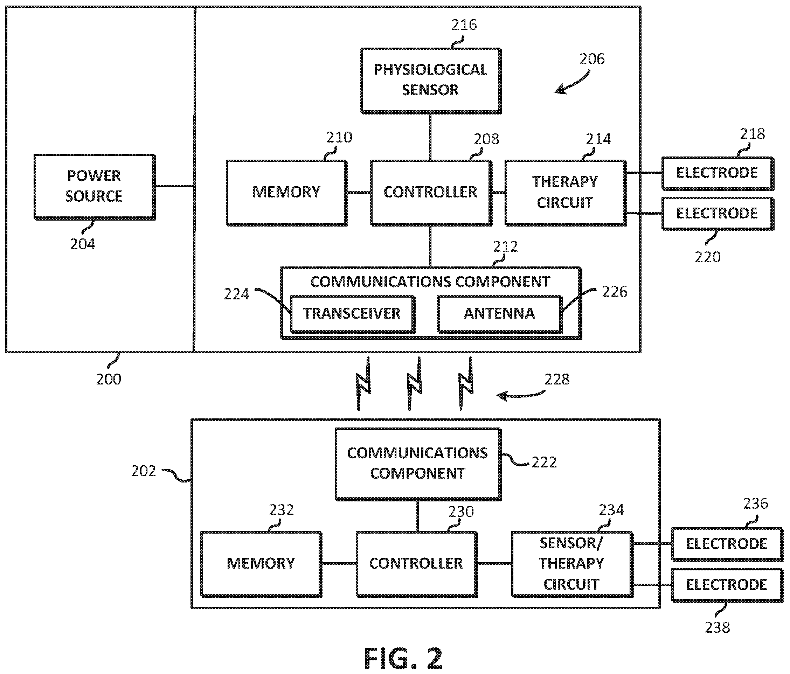

In certain instances, the IMD 102 has an internal receiver that may receive communications and/or energy from the controller 120, which may include a transmitter. The controller 120 may include a pulse generator that supplies an appropriate time-varying energy (e.g., current or voltage) to the IMD 102. The IMD 102 may include a power source for storing electrical energy, and may also have a triggering mechanism to deliver stored electrical energy to the adrenal gland. The IMD 102 may be a passive simulator such that stimulation energy is transmitted via the controller 120, stored with the IMD 102, and stimulated in response to a prompt from the controller 102. In other instances, the IMD 102 may be an active stimulator and provide stimulation based on control circuitry contained therein (*e.g.,* as described in further detail in FIGS. 2-3).

In certain instances, as noted above, multiple IMDs 102 may be implanted to apply stimulation to both adrenal glands 104, 106. The controller 120 may instruct delivery of the stimulation energy to one of the IMDs 102 to stimulate one of the adrenal glands 104, 106 at a time. In addition, the controller 120 may test between the IMDs 102 to determine which of the IMDs 102 achieves a desired response (*e.g.,* based on patient feedback and or measuring the levels of catecholamine or catecholamine metabolites in the patient). More specifically and in certain instances, one of the adrenal glands 104, 106 may respond better to the stimulation than the other one of the adrenal glands 104, 106. Thus, the controller 120 may altered delivery of the stimulation through the IMDs 102 to target one or both of the adrenal glands 104, 106 to achieve a desired response. In addition, the IMDs 102 may stimulate both the adrenal glands 104, 106 at the same time, or the stimulation applied thereto could be staggered (*e.g.,* according to a duty cycle). In certain instances, the coordinated stimulation of the adrenal glands 104, 106 by the IMDs 102 may reduce chances of attenuated effectiveness over time due to adaptation/tolerance/depletion of the adrenal glands 104, 106.

FIG. 2 is a block schematic diagram of a system including an IMD 200 (such as, for example, the IMD 102 depicted in FIG. 1) and a co-implanted device 202. As illustrated in FIG. 2, the IMD 200 includes a power source 204 that provides electrical energy to a set 206 of components. The set 206 of components include a controller 208, a memory 210, a communications component 212, a therapy circuit 214, and a physiological sensor 216. As shown in FIG. 2, the therapy circuit 214 is coupled to electrodes 218 and 220 and is configured to provide stimulation energy to the electrodes, which, in turn, provides the energy to a patient's body (e.g., an adrenal gland). The IMD 200 may include more than two electrodes. The power source 204 is configured to store electrical energy, and the physiological sensor 216 is powered by the power source 204, and coupled to the controller 208. The power source 204 may include one or more batteries, capacitors, and/or the like. In certain instances, the IMD 200 may include other components such as, for example, one or more sensor circuits (not shown), coupled to one or more sensors and/or the electrodes 218 and 220, for sensing physiological parameters.

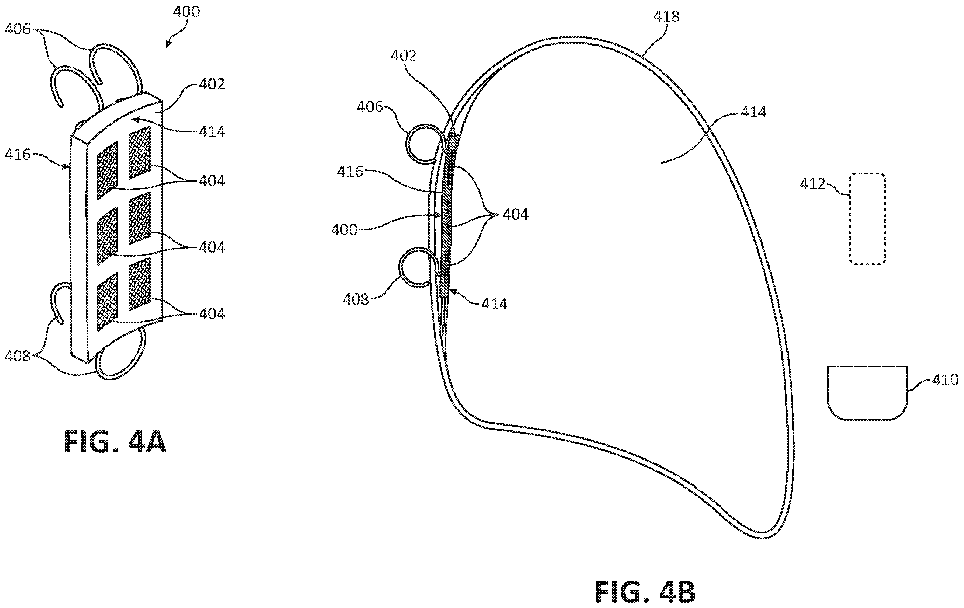

As described in further detail below (*e.g.,* with reference to FIG. 4), the physiological sensor 216 may be configured to capture one or more physiological signals. The physiological signals may include heart rate of the patient, heart rate variability of the patient (such as modulation in S1/S2 amplitudes with respiration), respiration rate of the patient, activity level of the patient, catecholamine levels of the patient (*e.g.,* chemosensor), metanephrine levels, metanephrine:creatinine (urine) ratio levels, body position, of the patient, body temperature of the patient, cardiac output of the patient, and arterial pressure of the patient, or any combination thereof (*e.g.,* heart rate / respiration rate ratio). The physiological sensor 216 may also measure other surrogates of catecholamine levels, or pain or non-pain symptoms of various disease states with neurohormonal or neurotransmitter dysfunction such as fibromyalgia (*e.g.,* as indicated by exhaustion), chronic fatigue, sleep apnea, or the like. A change in the above noted physiological responses may call for a modulation of the metabolization and exocytosis of catecholamines. Thus, the physiological sensor 216 is coupled to the controller 208, which may modulate stimulation energy, provided through the electrodes 218 and 220 via the therapy circuit 214.

The co-implanted device 202 includes a communications component 222 having circuits and one or more transmitters and/or receivers for communicating wirelessly with the IMD 200. The communications component 212 of the IMD 200 includes a transceiver 224 and an antenna 226 (or multiple antennae) that work together to facilitate wireless communication 228 with the co-implanted device 202, which may include one or more implantable co-implanted devices (e.g., the controller 120 depicted in FIG. 1). In certain instances, the communications component 212 may also facilitate communications with other IMDs 200 (e.g., other IMDs) such as, for example, to facilitate coordinated operations between the IMDs. The communications component 212 may include one or more transmitters, receivers, transceivers, transducers, and/or the like, and may be configured to facilitate any number of different types of wireless communication such as, for example, radio-frequency (RF) communication, microwave communication, infrared communication, acoustic communication, inductive communication, conductive communication, and/or the like. The communications component 222 may, in addition to facilitating wireless (e.g., RF, microwave, acoustic, etc.) communication with the IMD 200, facilitate wireless communication with an external device, such as a programming device, such that information may be provided to the co-implanted device 202 or supplied to the external device. In certain instances, the communications component 222 may include an antenna disposed on or in the co-implanted device 202 or on a distal portion of an attached lead (not shown).

In an implementation, the co-implanted device 202 includes a controller 230 that may include, for example, a processing unit, a pulse generator, and/or the like. The controller 230 may be a programmable micro-controller or microprocessor, and may include one or more programmable logic devices (PLDs) or application specific integrated circuits (ASICs). The controller 230 may execute instructions and perform desired tasks as specified by the instructions. The controller may also be configured to store information in the memory 232 and/or access information from the memory 232. The memory 232 may include volatile and/or non-volatile memory, and may store instructions that, when executed by the controller 230 cause methods and processes to be performed by the co-implanted device 202. For example, the controller 230 may process instructions and/or data stored in the memory 232 to control delivery of an electrical stimulation therapy by the IMD 200. Additionally, the co-implanted device 202 may sense physiological parameters and/or deliver therapy using a sensor/therapy circuit 234 that may be coupled, for example, to electrodes 236 and 238, a sensor (not shown), or a combination of these. In certain instances, the sensor/therapy circuit 234 may actually include multiple circuits. The memory 232 may be used to store stimulation parameters and sensed information according to some implementations. The co-implanted device 202 may also include a power source (e.g., a battery) (not shown) that supplies power to the circuits and components of the co-implanted device 202. In some implementations, the controller 230 may include memory as well. Although the present system is described in conjunction with a co-implanted device 202 having a microprocessor-based architecture, it will be understood that the co-implanted device 202 (or other device) may be implemented in any logic-based integrated circuit architecture, if desired.

The controller 230 may include digital-to-analog (D/A) converters, analog-to-digital (A/D) converters, timers, counters, filters, switches, etc. (not shown). The controller 230, sensor/therapy circuit 234 (e.g., sensing circuits), memory 232, and communications component 222 may work together to control communication between the co-implanted device 202 and one or more leadless electrode assemblies (e.g., IMD 200) to facilitate providing stimulation energy to the adrenal gland. In an implementation, the controller 230 may encode information, such as a unique identifier, stimulation energy threshold information, pulse width information, trigger signals, demand stimulation energy information, stimulation energy timing information, and the like, to be transmitted to the IMD 200.

Information from sense circuits included in the sensor/therapy circuit 234 may be used to adjust stimulation or communications parameters. The sense circuits may amplify and filter signals sensed from sensors coupled to one or both of the patient's adrenal glands. The sense circuits may include one or more A/D converters. In some implementations, the IMD electrodes that deliver simulation energy to the tissue are the same as the sense electrodes used to sense the local electrical voltage at the therapy site. In these cases, sensing may be paused when stimulation energy is being delivered. Similarly, the transmitter/receiver that receives communications may be the same as the transmitter/receiver that sends sensed information back to the co-implanted device 202, according to some implementations. In these cases, outgoing transmissions may be paused when communications are being received. The sensor/therapy circuit 234 of the co-implanted device 202 may include, for example, one or more can or housing electrodes disposed on an exterior surface of the co-implanted device 202.

The illustrative IMD 200 and co-implanted device 202 shown in FIG. 2 are not intended to suggest any limitation as to the scope of use or functionality of embodiments of the disclosure disclosed throughout this document. Neither should the illustrative IMD 200 and co-implanted device 202 be interpreted as having any dependency or requirement related to any single component or combination of components illustrated therein. For example, in embodiments, the illustrative IMD 200 may include additional components such as, for example, a sensor circuit (not illustrated) and may include the controller 208. Additionally, any one or more of the components depicted in FIG. 2 can be, in embodiments, integrated with various ones of the other components depicted therein (and/or components not illustrated). Any number of other components or combinations of components can be integrated with the illustrative IMD 200 and/or co-implanted device 202 depicted in FIG. 2, all of which are considered to be within the scope of this disclosure.

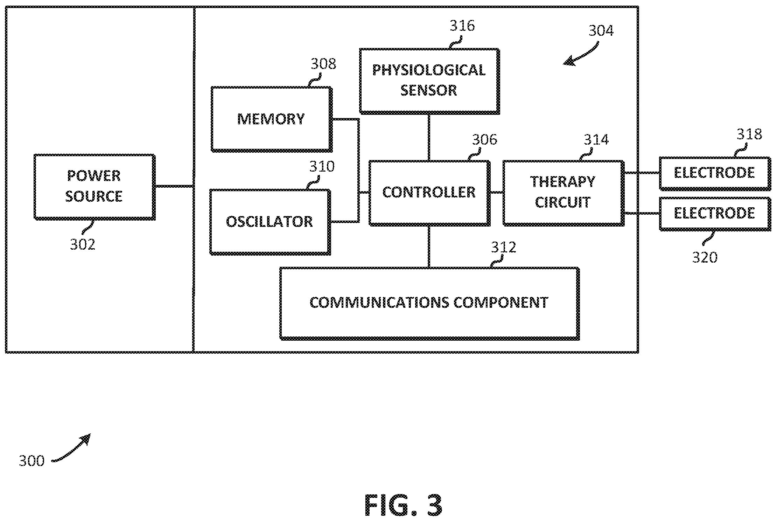

FIG. 3 is a schematic block diagram of an IMD 300 in accordance with aspects of embodiments of the disclosure. As shown in FIG. 3, the IMD 300 includes a power source 302 that powers operational circuitry 304 of the IMD 300. The operational circuitry 304 includes a controller 306 coupled to a memory 308. An oscillator 310 coupled to the controller 306 may be used as a clocking mechanism to provide timing functions to the controller 306. In certain instances, other types of clocking mechanisms may be used as well as, or in addition, to the oscillator 310. The operational circuitry 304 also includes a communications component 312, a therapy circuit 314, and a physiological sensor 316. As shown in FIG. 3, the therapy circuit 314 is coupled to electrodes 318 and 320 and is configured to provide stimulation energy to the electrodes, which, in turn, provide the energy to a patient's adrenal gland. The IMD 300 may include more than two electrodes. Additionally, the communications component 312 may be similar to the communications component 212 depicted in FIG. 2 and include a transceiver and an antenna. In certain instances, any number of the components illustrated in FIG. 3 may be, include, or be similar to, similarly named components depicted in FIG. 2. The IMD 300 may include any number of additional components as well such as, for example, a sensor circuit.

As described in further detail below (*e.g.,* with reference to FIG. 4), the physiological sensor 316 may be configured to capture one or more physiological signals. The physiological sensor 316 is coupled to the controller 306, which may modulate stimulation energy, provided through the electrodes 318 and 320 via the therapy circuit 314. The physiological signals may include heart rate of the patient, heart rate variability of the patient (such as modulation in S1/S2 amplitudes with respiration), respiration rate of the patient, activity level of the patient, catecholamine levels of the patient (*e.g.,* chemosensor), metanephrine levels, metanephrine:creatinine (urine) ratio levels, body position, of the patient, body temperature of the patient, cardiac output of the patient, and arterial pressure of the patient, or any combination thereof (*e.g.,* heart rate / respiration rate ratio). The physiological sensor 316 may also measure other surrogates of catecholamine levels, or pain or non-pain symptoms of various disease states. A change in the above noted physiological responses may call for a modulation of the metabolization and exocytosis of catecholamines.

FIG. 4A is an illustration of an example leadless implantable medical device 400 in accordance with embodiments of the disclosure. The leadless implantable medical device 400 may include a leadless body portion 402 configured to engage an adrenal gland 414 of a patient (shown in FIG. 4B). The leadless implantable medical device 400 may also include a plurality of electrodes 404, arranged with the leadless body portion 402. In addition, the leadless implantable medical device may communicatively be coupled to a controller 410 using wireless signals (as discussed in detail below). The controller 410 may be co-implanted with the leadless implantable medical device 400, or the controller 410 may be an external device that allows for patient input. In addition, the leadless implantable medical device 400 may be communicatively coupled to multiple controllers 410 or the leadless implantable medical device 400 may include the controller 410 (*e.g.,* as discussed above with reference to FIGs. 2-3).

One or more of the plurality of electrodes 404 may be configured to deliver stimulation energy to modulate catecholamine release from chromaffin cells within the adrenal gland 414 of the patient. Stimulation energy may be in the form of a pulsed electrical waveform to one or more of the plurality of electrodes 404 in accordance with a set of stimulation parameters. The leadless body portion 402 may include controller circuitry (as discussed in detail above with reference to FIGS. 2 and 3), which may be programmed therein, transmitted to controller circuitry included with the leadless body portion 402, transferred to the control circuitry, and/or the like. In addition, one or more of the plurality of electrodes 404 may be configured to sense the catecholamines released as a result of the delivered stimulation energy or configured to sense a biomarker affected by the delivered stimulation energy. The sensed level of catecholamines released may be provided as feedback to the controller circuitry, which may alter the stimulation energy to achieve a desired catecholamine release level.

Stimulation parameters may include, for example, electrode combinations that define which of the plurality of electrodes 404 that are activated as anodes (positive), cathodes (negative), turned on, turned off (zero), percentage of stimulation energy assigned to each electrode (fractionalized electrode configurations), and/or electrical pulse parameters, which define the pulse amplitude (measured in milliamps or volts depending on whether the controller circuitry supplies constant current or constant voltage to one or more of the plurality of electrodes 404), pulse duration (measured in microseconds), pulse rate (measured in pulses per second), pulse waveform, and/or burst rate (measured as the stimulation on duration X and stimulation off duration Y). The control circuitry may be capable of delivering the stimulation energy to the one or more of the plurality of electrodes 404 over multiple channels or over only a single channel. Stimulation energy may be used to identify therapy regions and/or to provide stimulation therapy to identified therapy regions or alter therapy regions over time to prevent exhaustion in one location.

In certain instances, the leadless implantable medical device 400 (via the controller circuitry) may be configured to intermittently or continuously instruct delivery of the stimulation energy through different combinations of the one or more of the plurality of electrodes 404. More specifically, the controller circuitry may instruct delivery of the stimulation energy through one or more of the plurality of electrodes 404 on a duty cycle based on a metabolization time of catecholamine. The duty cycle may include apply stimulation for 25% of a time period (*e.g.,* minutes, hours, or days), and withhold stimulation for 75% the time period (*e.g.,* minutes, hours, or days). Continuous stimulation through one or more of the plurality of electrodes 404 may not be necessarily required for reaching therapeutic levels of catecholamine. The duty cycle control of delivery of the stimulation energy may reduce battery consumption by the leadless implantable medical device 400 and reduces chances of attenuated effectiveness over time due to adaptation or depletion of the adrenal gland 414 (or adrenal glands if an additional leadless implantable medical device 400 is implanted on the patient's other adrenal gland). In addition, the stimulation energy may be delivered at a frequency between 2Hz and 20 kHz. The stimulation energy may be applied as bursts of energy include pulses at a frequency between 2Hz and 20 kHz or continuously (*e.g.,* 2Hz - 50 Hz, 50Hz - 100Hz, 100Hz - 500Hz, 500Hz - 1kHz, 1kHz - 5kHz, 5kHz - 10kHz, 10kHz - 20kHz or any combination thereof). In addition, a level of current applied may also be altered to change the stimulation energy. In addition, the frequency and/or pulse width stimulation energy may also be altered continuously or periodically altered over time. Altering the frequency and/or pulse width may reduce the chances of attenuated effectiveness of the stimulation over time due to adaptation of the adrenal gland 414. In any of these instances, the stimulation energy delivered may be altered in response to patient feedback based on the physical symptoms of the patient.

More specifically, the stimulation energy delivered may be altered in response to patient feedback based on the physical symptoms of the patient. Additionally, the controller 410 may instruct alteration of the stimulation energy provided to one or more of the plurality of electrodes 404 based on patient feedback. Therapy may be customized by calibrating to a target level of catecholamine release based on a change in physical symptoms or based on data obtained by the physiological sensor 412 and/or based on patient or physician input on an external device, communicatively coupled with the controller 410, that may control the stimulation energy level. The patient may be able to activate / deactivate therapy correlating to his/her own perceptions of their symptoms (*e.g.,* when they feel symptoms of chronic fatigue, they may activate therapy). In these scenarios, the controller 410 may include a self-limiting mechanism to avoid depletion of catecholamines (*e.g.,* the controller 410 down-regulates or turns off stimulation to after some period of time), so that a patient cannot cause depletion of catecholamines by requesting constant therapy.

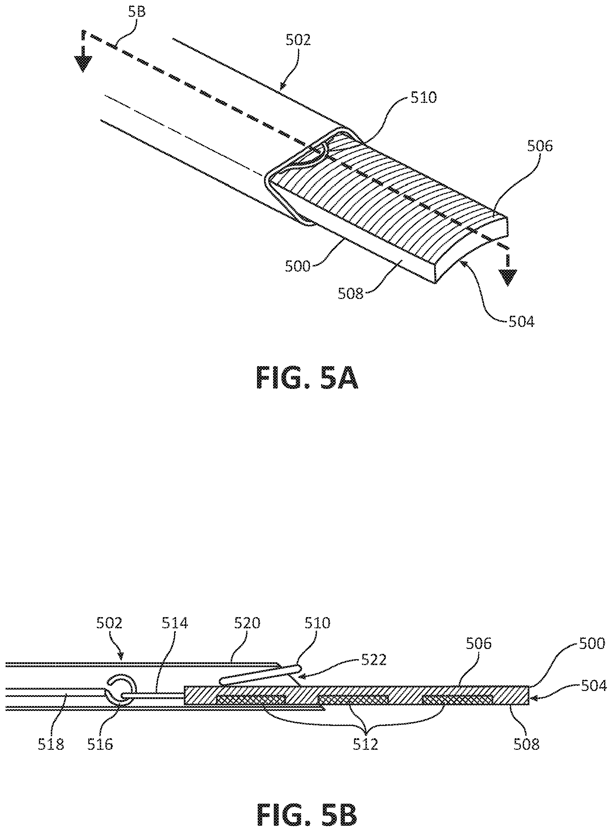

In certain instances, the leadless implantable medical device 400 may include a mechanical attachment structure arranged with the leadless body portion 402 that is configured to engage the adrenal gland 414 (or periadrenal connective tissue). As shown in FIG. 4A, the mechanical attachment structure includes a first set of talons 406 and a second set of talons 408. In certain embodiments, the leadless implantable medical device 400 may include a single talon, a single set of talons, or additional talons. In addition, the mechanical attachment structure may also comprises a helical structural (such as a rigid helix) or a barb structure (*e.g.,* as shown in FIGS. 5A-B).

The leadless body portion 402 includes a first side 414 and a second side 416. In certain instances, the mechanical attachment structure arranged with the leadless body portion 402 is arranged one of the first side 414 or the second side 416, and the plurality of electrodes 404 are arranged on the other of the first side 414 or the second side 416. More specifically and as shown in FIG. 4A, the first set of talons 406 and the second set of talons 408 (collectively a plurality of talons) are arranged on the first side 414 of the leadless body portion 402 and the plurality of electrodes 404 are arranged on the second side 416 of the leadless body portion 402. The first set of talons 406 and the second set of talons 408 may be collapsed toward the second side 416 of the leadless body portion 402 to a delivery configuration in response to retraction within a deployment system (*e.g.,* as shown in further detail in FIGS. 5A-B) during loading of the leadless implantable medical device 400 therein or for repositioning of the leadless implantable medical device 400. The first set of talons 406 and the second set of talons 408 may spring open to a deployed configuration (shown in FIGS. 4A-B).

The mechanical attachment structure and the plurality of electrodes 404 may be arranged on the same side of the leadless body portion 402. In addition and as shown, the first set of talons 406 and the second set of talons 408 may be curved in common direction. In other instances, the first set of talons 406 may be curved in a first direction, and the second set of talons 408 may be curved in a second direction. The first direction and the second direction may be opposites (*e.g.,* 180 degrees relative to one another). In other instances, the first direction may be perpendicular to the second direction. In addition, the first set of talons 406 may have one talon curved in the first direction and another in the second direction, and the second set of talons 408 may have one talon curved in the first direction and another in the second direction.

The leadless implantable medical device 400 may also include a physiological sensor 412 that configured to measure at least one physiological parameter, and alter the stimulation energy in response thereto. Although shown as a separate element in FIG. 4B, the physiological sensor 412 may be incorporated with the leadless body portion 402. In other instances, the physiological sensor 412 may be a remote implantable or wearable monitoring system (in communication with the controller 410 and the leadless body portion 402). The physiological sensor 412 may also be a separate sensing lead that connects into the controller 410. The separate sensing lead may monitors signals from another target away from an adrenal gland of a patient to capture one or more physiological signals (*e.g.,* indicative of blood pressure). In addition, more than physiological sensor 412 may be used.

FIG. 4B is an illustration of the leadless implantable medical device 400, shown in FIG. 4A, attached to the adrenal gland 414 in accordance with embodiments of the disclosure. More specifically, the leadless implantable medical device 400 may configured to engage a capsule or pericapsular connective tissue 418 of the adrenal gland 414. The capsule 418 surrounds the adrenal gland 414. The leadless implantable medical device 400 may be attached to an exterior surface of the capsule 418, within the capsule 418, an interior surface of the capsule 418 (*e.g.,* between the capsule and the remaining portions of the adrenal gland 414) or to the renal fascia, Gerota's fascia, within the perirenal fat, within the periphrenic space or anterior pararenal space.

As shown in FIG. 4B, the plurality of electrodes 404 are arranged inwardly toward the adrenal gland 414, and the first set of talons 406 and the second set of talons 408 are arranged outwardly therefrom. In addition and as shown, the leadless implantable medical device 400 is shown implanted between the capsule 418 and the adrenal gland 414. The first set of talons 406 and the second set of talons 408 may puncture and protrude through the capsule 418 (*e.g*., renal fascia, Gerota's fascia, the perirenal fat, the periphrenic space or the anterior pararenal space) and mitigate against migration of the leadless implantable medical device 400. In other instances, the first set of talons 406 and/or the second set of talons 408 may be arranged on the second side 414 of the leadless body portion 402 (the same side of the leadless body portion 402 as the plurality of electrodes 404). As a result, the first set of talons 406 and/or the second set of talons 408 may puncture the adrenal gland 414. The first set of talons 406 and/or the second set of talons 408 may puncture the adrenal gland 414 through a portion or the entirety of the capsule 418, or directly puncture the adrenal gland 414. In addition, one or more of the first set of talons 406 and/or the second set of talons 408 may be configured as an electrode. Thus, the one or more of the first set of talons 406 and/or the second set of talons 408, configured as an electrode, may be configured with the plurality of electrodes 404 to deliver stimulation energy to modulate catecholamine release from chromaffin cells within the adrenal gland 414 of the patient. In other instances, the first set of talons 406 and/or the second set of talons 408 may be formed Nitinol (NiTi) or from an electrically conductive and biocompatible metal or polymer (when configured as an electrode).

In certain instances, the leadless implantable medical device 400 may include a communications component/circuitry (*e.g.,* a described above with reference to FIGS. 2-3) configured to communicate wireless signals. The communications component/circuitry allows the leadless implantable medical device 400 to communicate with a co-implanted device such as a controller 410. In certain instances, the physiological sensor 412 may be a separate co-implanted device. The leadless implantable medical device 400, the controller 410, and the optional separate physiological sensor 412 may form an adrenal gland stimulation system.

In embodiments, the wireless signals between the leadless implantable medical device 400, the controller 410, and the physiological sensor 412 may be, or include, communicated over a wireless communication link such as, for example, a short-range radio link, such as Bluetooth, IEEE 802.11, a proprietary wireless protocol, and/or the like. In embodiments, for example, the communication link may utilize Bluetooth Low Energy radio (Bluetooth 4.1), or a similar protocol, and may utilize an operating frequency in the range of 2.40 to 2.48 GHz. The term "communication link" may refer to an ability to communicate some type of information in at least one direction between at least two devices, and should not be understood to be limited to a direct, persistent, or otherwise limited communication channel. That is, according to embodiments, a communication link may be a persistent communication link, an intermittent communication link, an ad-hoc communication link, and/or the like. A communication link may refer to direct communications between one or more devices, and/or indirect communications that travel between the one or more devices via at least one other device (e.g., a repeater, router, hub, and/or the like). A communication link may facilitate uni-directional and/or bi-directional communication between the linked devices.

Similar to the leadless implantable medical device 400, the co-implanted device 410 includes a communications component having circuits and one or more transmitters and/or receivers for communicating wirelessly with the leadless implantable medical device 400 (as discussed in further detail with reference to FIGS. 2-3). The co-implanted device 410 may be configured to transmit data via the wireless signals instructing at least one of the plurality of electrodes 404 to deliver the stimulation energy. The co-implanted device 410 may instruct at least one of the plurality of electrodes 404 to deliver the stimulation energy based on a duty cycle based on a metabolization time of catecholamine. In other instances, the implanted device 410 may instruct at least one of the plurality of electrodes 404 to deliver the stimulation energy at a frequency between 2Hz and 20 kHz (*e.g.,* 2Hz - 50 Hz, 50Hz - 100Hz, 100Hz - 500Hz, 500Hz - 1kHz, 1kHz - 5kHz, 5kHz - 10kHz, 10kHz - 20kHz or any combination thereof).

The physiological sensor 412 may also wirelessly communicate with the leadless implantable medical device 400 and the controller 410, and the physiological sensor 412. In certain instances, the physiological sensor 412 may be configured to transmit data via the wireless signals altering the stimulation energy in response to the measurement of the physiological sensor 412.

In certain instances, the physiological sensor 412 may be configured to measure at least one of: heart rate of the patient, heart rate variability of the patient (such as modulation in S1/S2 amplitudes with respiration), respiration rate of the patient, activity level of the patient, catecholamine levels of the patient (*e.g.,* chemosensor), metanephrine levels, metanephrine:creatinine (urine) ratio levels, body position, of the patient, body temperature of the patient, cardiac output of the patient, and arterial pressure of the patient, or any combination thereof (*e.g.,* heart rate / respiration rate ratio). The activity level of the patient may assist in informing the leadless implantable medical device 400, directly or indirectly through the controller 412, whether stimulation energy should be altered (*e.g.,* increased) due to an increase in the circulation and uptake of catecholamines. In certain instances, the physiological sensor 412 may be implanted in the bladder or other areas of the urinary tract (e.g. renal pelvis, ureter) and determine the urine metanephrine:creatinine levels, which can be a surrogate for plasma epinephrine and norepinephrine levels. A change in the above noted physiological responses may require a modulation the exocytosis or metabolization of catecholamines, for a resultant change in the physiological responses necessary. As a result, the controller 412 may alter (increase or decrease) the stimulation energy to alter or modulate the release of catecholamines from the adrenal gland 414 to maintain a desired level of catecholamines in the patient.

In certain instances, the physiological sensor 412 may be configured to measure the body temperature of the patient. The physiological sensor 606 may also be configured to measure the cardiac output, stroke volume of the heart (*e.g.,* S1 indicative of contractility or S2 as indicative of blood pressure changes due to sympathetic activity) contractility body of the heart, and/or mean arterial pressure/pulse pressures/systolic/diastolic of the patient (via intra-arterial or peri-arterial approaches). The physiological sensor 412 may also measure other surrogates of catecholamine levels, or pain or non-pain symptoms of various disease states with neurohormonal or neurotransmitter dysfunction such as fibromyalgia (*e.g.,* as indicated by exhaustion), chronic fatigue, sleep apnea, or the like. In certain instances, the physiological sensor 606 may be configured to measure surrogates of catecholamine levels associated with Postural Orthostatic Tachycardia Syndrome (POTS), orthostatic hypotension (OH), orthostatic intolerance (OI), or surrogates of catecholamine levels associated with heart failure and migraines, all which have a cause and effect due to changes in circulating catecholamine levels. The physiological sensor 412 may also measure decreased positive expiratory pressure (PEP), decreased raw left ventricular ejection time (LVET) and/or increased LVET corrected for heart rate. A change in the above noted physiological responses may call for a modulation of the metabolization and exocytosis of catecholamines. As a result, the controller 410 may modulate the stimulation energy to modulate the release of catecholamines from the adrenal gland 414 to maintain a desired level of catecholamines in the patient.

This type of coordinated stimulation may provide a closed-loop system which uses markers of systemic catecholamines or disease state symptoms to optimize therapy. In certain instances, two or more physiological parameters to provide the leadless implantable medical device 400 with closed-loop control and enable predictive power to provide therapy only when needed or otherwise enabling improved therapy titration (e.g. stimulation amplitude, charge, duration) and optimize clinical outcomes. To address either steady state deficiency or excess or a transient deficiency or excess (ability to ramp up/down quickly) of the adrenal gland 414 (or glands), the system may use more than one physiological sensor 412 that communicates with the leadless implantable medical device 400, directly or indirectly through the controller 410 as described above. The sensors may provide additional quantification, measurement, and tracking and trend analysis of the catecholamine levels or metabolites or disease status as a function of activity to optimize therapy. The measurements of the various physiological sensors may be collected by the leadless implantable medical device 400 and/or the controller 412 aggregated to alter delivery of the stimulation energy provided through the plurality of electrodes 404. In certain instances, the controller 412 may be programmed based on patient customization. For example, a patient may prefer stimulation energy that is associated with lower systemic catecholamines. Thus, the controller 412 may program the stimulation to iterate in this manner. Another patient may prefer a state with higher systemic catecholamine, which would result in different stimulation energy (*e.g.,* as compared to a patient that prefers lower systemic catecholamines). The controller 412 may be programmed with references within patients such that the stimulation energy may change based on activity level and time of day (awake/sleeping). Biomarker data could be used to calibrate the patients' preferences (increase or decrease) in systemic catecholamine levels in a closed loop system on a patient-by-patient basis.

The physiological sensor 412 may measure the steady state deficiency or excess of the catecholamines levels of the patient by determining the physiological response measured by the physiological sensor 412 at a given steady state activity level. A transient deficiency or excess may be monitored by looking at "transient" physiological sensor 412 measurements around activity state transition. In certain instances, a desirable response will be quick and more like an "underdamped" system with respect to maintaining the catecholamines levels of the patient, whereas a poor performance would look like an over-damped system.

As a specific example, the physiological sensor 412 may be configured to monitor heart rate activity. In the patient, the physiological sensor 412 may continuously record heart rate (HR) and activity (movement) information (e.g., using an accelerator), or body position (*e.g.,* orthostatic imbalance) and parse the data stream into discrete activity bands. The time axes are separated in terms of transitions between the activity bands. HR data around the transitions may yield metrics for a transient deficiency, whereas HR data from in-between transitions and sufficiently away from the transitions may yield data for a steady state deficiency. In certain instances, in place of or along with monitoring the heart rate of the patient, the physiological sensor 412 may measure heart sounds. The heart sounds may provide an indication as to whether the patient is awake or asleep to influence modulation of catecholamine levels. More specifically, when the patient is sleeping, the controller 410 may be programmed to optimize (decrease) catecholamine levels to avoid insomnia by decreasing stimulation levels provided through the plurality of electrodes 404 as compared to when the patient is awake. The stimulation parameters may also be maintained to ensure catecholamine levels stay above a lower limit (low circulating catecholamine levels may be associated with a patient's current challenges with inability to sleep) or below an upper limit. In addition, when the patient is exercising, the controller 410 may ensure optimal levels to reduce pain and fatigue (*e.g.,* modulate levels based on baselines changes to exertion or stress) and ensure catecholamine levels stay above a lower limit (higher circulating levels may be associated with these patients' current challenges with lower tolerance for prolonged activity). As a result, the controller 410 may be configured to ensure appropriate modulation of catecholamine levels, which may vary when the patient is exercising, at rest or undergoing stressful experiences.

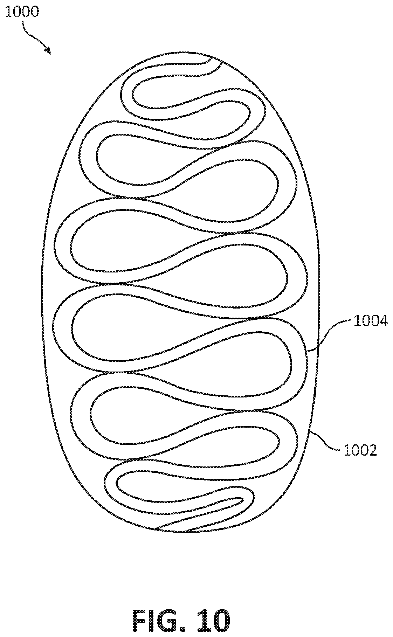

The illustrative components shown in FIGS. 4A-B are not intended to suggest any limitation as to the scope of use or functionality of embodiments of the disclosed subject matter. Neither should the illustrative components be interpreted as having any dependency or requirement related to any single component or combination of components illustrated therein. Additionally, any one or more of the components depicted in any of the FIGS. 4A-B may be, in embodiments, integrated with various other components depicted therein (and/or components not illustrated), all of which are considered to be within the ambit of the disclosed subject matter. For example, the leadless implantable medical device 400 may include the circuitry described with reference to FIGS. 2-3. In addition, the leadless implantable medical device 400 may be deployed using the systems described with reference to FIGS. 5A-B. Further, the leadless implantable medical device 400 may include any of the mechanical attachment mechanisms described with reference to FIGS. 5-9 in place of or in addition to the first set of talons 406 and the second set of talons 408, and the plurality of electrodes 404 may have single or dual electrode configuration, as described with reference to FIGS. 1-3, or a coiled structure as described with reference to FIG. 10.

FIG. 5A is an illustration of an example leadless implantable medical device 500 and a deployment system 502 in accordance with embodiments of the disclosure. The leadless implantable medical device 500 may include a leadless body portion 500 configured to engage an adrenal gland, adrenal capsule, or pericapsular connective tissue of a patient. The leadless implantable medical device 500 may be configured to attach to the adrenal gland, adrenal capsule, or pericapsular connective tissue via a mechanical attachment structure. As shown in FIG. 5A, the mechanical attachment structure, in certain embodiments, is a barb 510 arranged on a first side 506 of the leadless body portion 500. Although a single barb 510 is shown, the leadless body portion 500 may have multiple barbs 510 or other similar mechanical attachment structures. The leadless implantable medical device 500 may also include a plurality of electrodes 512 (shown in FIG. 5B), arranged on a second side 508 of the leadless body portion 500. One or more of the plurality of electrodes 512 may be configured to deliver stimulation energy to modulate catecholamine release from chromaffin cells within the adrenal gland of the patient. The deployment system 502 may be configured to deliver the leadless implantable medical device 500 to the adrenal gland of the patient. In addition, the barb 510 may be configured as an electrode. Thus, the barb 510, when configured as an electrode, may be configured with the plurality of electrodes 512 to deliver stimulation energy to modulate catecholamine release from chromaffin cells within the adrenal gland of the patient. The barb 510 may be formed Nitinol (NiTi) or from an electrically conductive and biocompatible metal or polymer (when configured as an electrode).

FIG. 5B is a cross-sectional illustration of the example leadless implantable medical device 500 and the deployment system 502 shown in FIG. 5A in accordance with embodiments of the disclosure. As shown in FIG. 5B, the deployment system 502 may be releasably coupled to the leadless implantable medical device 500 by way of a loop structure 514, arranged with the leadless implantable medical device 500, and a hook structure 516. The hook structure 516 may be attached to a deployment line 518. The hook structure 516 and the hook structure 516 may be manipulated by a physician to deploy the leadless implantable medical device 500. The deployment system 502 may also include a sheath 520. The sheath 520 includes an opening 522 through which the leadless implantable medical device 500 is deployed. During the deployment procedure (*e.g.,* laparoscopic or percutaneous procedure, which may be a retroperitoneal approach), the sheath 522 is routed to the adrenal gland of the patient. The physician may push the leadless implantable medical device 500 out of the sheath 520 by manipulating the deployment line 518.

After the leadless implantable medical device 500 is at least partially deployed from the sheath 520 such that the barb 510 extends out of the opening 522, the physician may manipulate the deployment line 518 to advance the leadless implantable medical device 500 toward the adrenal gland, and engage the barb 510 therewith. In certain instances, the barb 510 may be attached to periadrenal connective tissue, an exterior surface of the capsule, within the capsule, an interior surface of the capsule (*e.g.,* between the capsule and the remaining portions of the adrenal gland). The barb 510 may be pressed toward the first side 506 of the leadless implantable medical device 500 and collapsed in a delivery configuration, and spring open in a deployed configuration (as shown in FIG. 5B) in response to the leadless implantable medical device 500 being advanced out of the opening 522.

The opening 522 of the sheath 520 may have a taper (*e.g.,* as shown in FIG. 5A) configured to puncture the capsule and position the leadless implantable medical device 500 therein. The physician may use dynamic ultrasound to assist in placement of the leadless implantable medical device 500. The plurality of electrodes 512, the barb 510, or other portions of the leadless implantable medical device 500 may be radiopaque to assist in visualizing the leadless implantable medical device 500. After the leadless implantable medical device 500 is engaged with the adrenal gland or periadrenal connective tissue, the deployment system 502 may be removed from the patient's body. In instances where multiple leadless implantable medical devices 500 are implanted (*e.g.,* on each adrenal gland), the physician may load another of the leadless implantable medical devices 500 within the deployment system 502. The deployment system 502 may be advanced toward the target location (*e.g.,* the other adrenal gland) and the additional leadless implantable medical device 500 may be implanted.

In certain instances, the deployment system 502 may be used for repositioning or removal of an implanted leadless implantable medical device 500. As shown in FIG. 5B, the deployment line 518 and the hook structure 516 may be releasably coupled to the loop structure 514 arranged with the leadless implantable medical device 500. The hook structure 518 may be used to engage an implanted leadless implantable medical device 500, and the physician may retract the leadless implantable medical device 500 into the deployment system 502 by manipulating the deployment line 518 (which may extend along the sheath 520 to the physician outside of the patient). In retracting the leadless implantable medical device 500 into the deployment system, the barb 510 may be collapsed from the deployed configuration (shown in FIG. 5B) toward in the delivery configuration by contacting the opening 522 of the sheath 520.

The illustrative components shown in FIGS. 5A-B are not intended to suggest any limitation as to the scope of use or functionality of embodiments of the disclosed subject matter. Neither should the illustrative components be interpreted as having any dependency or requirement related to any single component or combination of components illustrated therein. Additionally, any one or more of the components depicted in any of the FIGS. 5A-B may be, in embodiments, integrated with various other components depicted therein (and/or components not illustrated), all of which are considered to be within the ambit of the disclosed subject matter. For example, the deployment system 502 may be used to deploy any of the leadless implantable medical devices discussed herein.

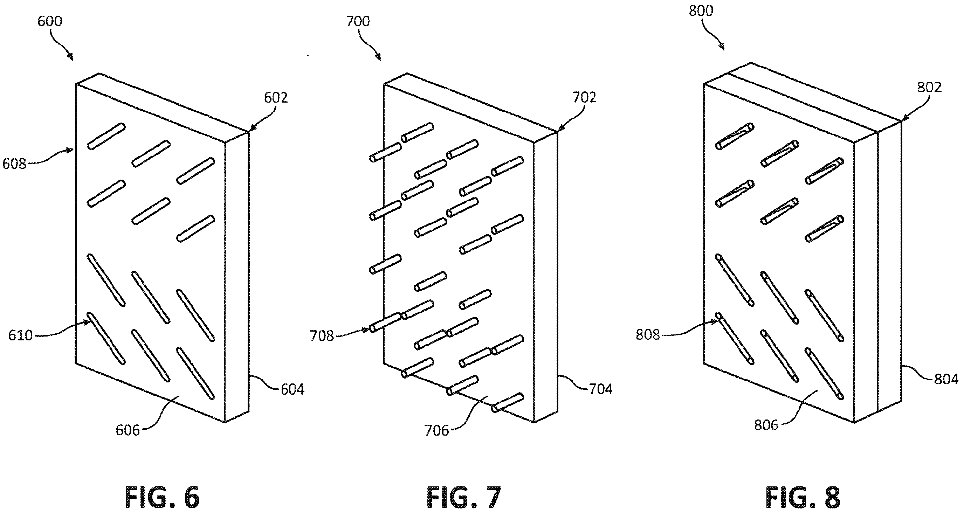

FIG. 6 is an illustration of an example leadless implantable medical device 600 having a mechanical attachment structure in accordance with embodiments of the disclosure. The leadless implantable medical device 600 includes a leadless body portion 602 having a first side 604 and a second side 606, with the mechanical attachment mechanism arranged on either one or both of the first side 604 and the second side 606.

As shown in FIG. 6, the mechanical attachment mechanism includes a first set of protrusions 608 and a second set of protrusions 610 that are configured to attach to an adrenal gland of a patient. In certain instances, the first set of protrusions 608 and the second set of protrusions 610 may be configured to attach to an exterior surface of a capsule or pericapsular connective tissue (surrounding the adrenal gland), within the capsule, an interior surface of the capsule (*e.g.,* between the capsule and the remaining portions of the adrenal gland), or cortex of the adrenal gland.

The first set of protrusions 608 and the second set of protrusions 610 (forming a plurality of protrusions) may be angled relative to the second side 606 of the leadless body portion 602. The first set of protrusions 608 and the second set of protrusions 610 may be angled in a common direction or in different directions. In certain instances, the first set of protrusions 608 and the second set of protrusions 610 may be angled toward the center of the leadless body portion 602, and in other instances, one of the first set of protrusions 608 and the second set of protrusions 610 may be angled toward the center of the leadless body portion 602, and the other of the first set of protrusions 608 and the second set of protrusions 610 angled toward a top of the leadless body portion 602. In certain instances, the first set of protrusions 608 and the second set of protrusions 610 may arranged at an angle between greater than 0 and less than 90 degrees relative to the second surface 606. In addition, the first set of protrusions 608 and the second set of protrusions 610 may be arranged on the first side 604 of the leadless body portion 602 in addition to or alternatively from being arranged on the second side 606. Further, one set of the first set of protrusions 608 and the second set of protrusions 610 may be arranged on the first side 604 or the second side 606 and the other of the first set of protrusions 608 and the second set of protrusions 610 may be arranged on the other of the first side 604 or the second side 606. Although the leadless body portion 602 is rectangular in shape, other shapes are contemplated (*e.g.,* oblong, cylindrical).

Although not shown, the leadless implantable medical device 600 may include one or more electrodes, arranged on the first side 604 and/or the second side 606, that are configured to deliver stimulation energy to modulate catecholamine release from chromaffin cells within the adrenal gland. More specifically, the leadless implantable medical device 600 may be configured to target a therapy location that includes a portion of an adrenal gland that is identified as being likely to be associated with an adrenal gland condition (*e.g.,* disorders such as chronic fatigue syndrome, fibromyalgia, orthostatic intolerance, irritable bowel syndrome (IBS), Crohn's disease, mood disorders/depression, or other disease states with neurotransmitter or neurohormonal dysfunction).

FIG. 7 is an illustration of an example leadless implantable medical device 700 having another example mechanical attachment structure in accordance with embodiments of the disclosure. The leadless implantable medical device 700 includes a leadless body portion 702 having a first side 704 and a second side 706. As shown in FIG. 7, the mechanical attachment mechanism includes a plurality of protrusions 708 that are configured to attach to an adrenal gland of a patient. In certain instances, the plurality of protrusions 708 may be configured to attach to an exterior surface of a capsule (surrounding the adrenal gland), within the capsule, or an interior surface of the capsule (*e.g.,* between the capsule and the remaining portions of the adrenal gland).

The plurality of protrusions 708 may be perpendicular to the second side 706 of the leadless body portion 702. In addition, the plurality of protrusions 708 may be arranged on the first side 704 of the leadless body portion 702 in addition to or alternatively from being arranged on the second side 706. In addition and although not shown, the leadless implantable medical device 700 may include one or more electrodes, arranged on the first side 704 and/or the second side 706, that are configured to deliver stimulation energy to modulate catecholamine release from chromaffin cells within the adrenal gland.

FIG. 8 is an illustration of an example leadless implantable medical device having another example mechanical attachment structure in accordance with embodiments of the disclosure. The leadless implantable medical device 800 includes a leadless body portion 802 having a first side 804 and a second side 806. As shown in FIG. 8, the mechanical attachment mechanism includes a roughed surface 808 that is configured to attach to an adrenal gland of a patient. In certain instances, the roughed surface 808 may be configured to attach to an exterior surface of a capsule (surrounding the adrenal gland), within the capsule, or an interior surface of the capsule (*e.g.,* between the capsule and the remaining portions of the adrenal gland).

The roughed surface 808 may be formed by score marking or otherwise altering the second side 706 of the leadless body portion 702. The roughed surface 808 may be formed arranging divots in the second side 706 of the leadless body portion 702. In addition, the roughed surface 808 may be arranged on the first side 704 of the leadless body portion 702 in addition to or alternatively from being arranged on the second side 706. In addition and although not shown, the leadless implantable medical device 800 may include one or more electrodes, arranged on the first side 804 and/or the second side 806, that are configured to deliver stimulation energy to modulate catecholamine release from chromaffin cells within the adrenal gland. In other instances, in addition to or in place of the mechanical attachment mechanisms, tissue glue, an adhesive, and/or a hydrogel or hydrogel/polymer type may be applied to the leadless implantable medical device 800 for adhesive attachment of the leadless implantable medical device 800 to the adrenal gland.

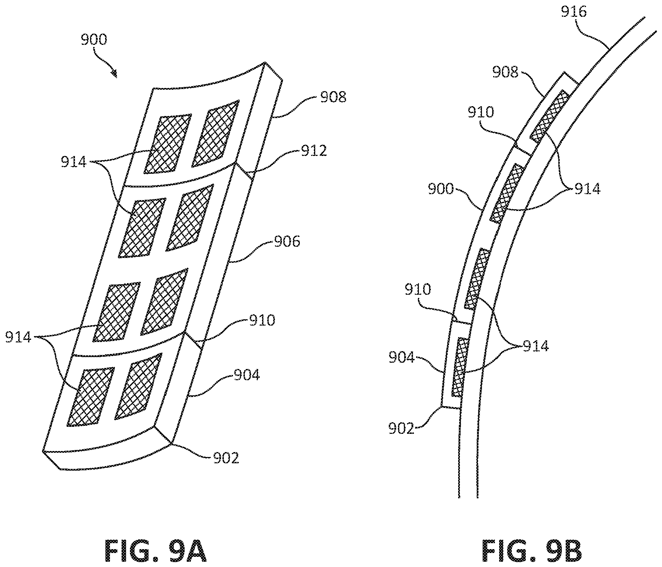

FIG. 9A is an illustration of an example leadless implantable medical device 900 including a leadless body portion 902 having flexible portions in accordance with embodiments of the disclosure. The leadless body portion 902 may have a first end section 904, a second end section 908, and an intermediate section 906 therebetween. The first end section 904 and the second end section 908 may have a greater flexibility than the intermediate section 906. Transitions 910, 912 between the intermediate section 906 and the first end sections 904 and the intermediate section 906 and the second end section 908 may allow the leadless body portion 902 to curve and conform around a patient's adrenal gland 916 (as shown in FIG. 9B). The differences in rigidity of the first end section 904 and the second end section 908 (more flexible) and the intermediate section 906 (less flexible) force the leadless body portion 902 to curve at the transitions 910, 912 and conform to the adrenal gland 916.

In certain instance, the leadless implantable medical device 900 may include a barb(s), a rigid helix(s), and/or a talon(s) or other mechanical attachment mechanisms discussed herein to further enhance the attachment leadless implantable medical device 900 to the adrenal gland 916. In other instances, in addition to or in place of the mechanical attachment mechanisms, suture, tissue glue, an adhesive, and/or a hydrogel may be applied to the leadless body portion 902 for adhesive attachment to the adrenal gland 916.

In addition, the leadless implantable medical device 900 may include plurality of electrodes 914. The plurality of electrodes 914 may be configured to deliver stimulation energy to modulate catecholamine release from chromaffin cells within the adrenal gland. More specifically, the leadless implantable medical device 900 may be configured to target a therapy location that includes a portion of an adrenal gland that is identified as being likely to be associated with an adrenal gland condition (*e.g.,* disorders such as chronic fatigue syndrome, fibromyalgia, orthostatic intolerance, irritable bowel syndrome (IBS), Crohn's disease, mood disorders/depression, or other disease states with neurotransmitter or neurohormonal dysfunction).

FIG. 9B is an illustration of the example leadless implantable medical device 900, shown in FIG. 9A, arranged with (and conforming to) a portion the patient's adrenal gland 916 in accordance with embodiments of the disclosure. The leadless implantable medical device 900 may be attached within the capsule that surrounds the adrenal gland 916, or to an interior surface of the capsule (*e.g.,* between the capsule and the remaining portions of the adrenal gland 916) or periadrenal connective tissue, which forces the leadless body portion 902 to conform to the adrenal gland 916. The leadless implantable medical device 900 may also be submuscularly placed adjacent to the adrenal gland 916 (*e.g.,* at or adjacent to the epaxial muscles). As a result, the first end section 904 and the second end section 908 of the leadless body portion 902 may be configured to enhance engagement of the adrenal gland 916 for mechanical attachment thereto. In addition, one or more of the first end section 904, the second end section 908, and the intermediate section 906 may have shape memory properties such that the leadless body portion 902 curves to conform to the adrenal gland 916, and remains in the curved configuration (shown in FIG. 9B). This may allow the leadless body portion 902 to capture a surface area of the adrenal gland 916, which may enhance the depth of stimulation applied by the plurality of electrodes 914.

FIG. 10 is an illustration of another example leadless implantable medical device 1000 in accordance with embodiments of the disclosure. The leadless implantable medical device 1000 may include a leadless body portion 1002 and a patch electrode 1004. The patch electrode 1004 may have a coiled structure forming a plurality of individually insulated electrodes. The patch electrode 1004 may be arranged on the leadless body portion 1002 by metal injection moulding, screen printing, or other printed circuit board manufacturing mechanisms. The patch electrode 1004 may be arranged using a laparoscopic procedure. Edges of the patch electrode 1004 may include tissue glue, an adhesive, and/or a hydrogel or hydrogel/polymer type for attachment of the patch electrode 1004 to the adrenal gland.

In certain instances, the patch electrode 1004 may be configured to deliver stimulation energy to modulate catecholamine release from chromaffin cells within an adrenal gland of a patient. More specifically, the leadless implantable medical device 1000 may be configured to target a therapy location that includes a portion of an adrenal gland that is identified as being likely to be associated with an adrenal gland condition (*e.g.,* disorders affecting the motor system such as chronic fatigue syndrome, fibromyalgia, orthostatic intolerance, irritable bowel syndrome (IBS), Crohn's disease, mood disorders/depression, or other disease states with neurotransmitter or neurohormonal dysfunction). In certain instances, the patch electrode 1004 may curve and conform to surfaces. Thus, the patch electrode 1004 may enhance conforming to the adrenal gland.

The illustrative components shown in FIGS. 6-10 are not intended to suggest any limitation as to the scope of use or functionality of embodiments of the disclosed subject matter. Neither should the illustrative components be interpreted as having any dependency or requirement related to any single component or combination of components illustrated therein. Additionally, any one or more of the components depicted in any of the FIGS. 6-10 may be, in embodiments, integrated with various other components depicted therein (and/or components not illustrated), all of which are considered to be within the ambit of the disclosed subject matter. For example, the leadless implantable medical devices 600, 700, 800, 900, 1000 may include the circuitry described with reference to FIGS. 2-3 and may be coupled to a controller (as described with reference to FIGS. 1-2 and 4) and/or physiological sensor (described with reference to FIG. 4). In addition, the leadless implantable medical devices 600, 700, 800, 900, 1000 may be deployed using the systems described with reference to FIGS. 5A-B.

Various modifications and additions can be made to the exemplary embodiments discussed without departing from the scope of the present disclosure. For example, while the embodiments described above refer to particular features, the scope of this disclosure also includes embodiments having different combinations of features and embodiments that do not include all of the described features. Accordingly, the scope of the present disclosure is intended to embrace all such alternatives, modifications, and variations as fall within the scope of the claims, together with all equivalents thereof.

## Claims

1. An implantable medical device (102, 400, 500, 600, 700, 800, 900, 1000) for delivering leadless stimulation therapy to an adrenal gland of a patient comprising:
a leadless body portion (116, 402, 504, 602,702, 802, 902, 1002) configured to engage an adrenal gland (104, 414, 916) or periadrenal connective tissue (418) of a patient; and
at least one electrode (122, 404, 512, 914,1004) arranged with the leadless body portion and configured to deliver stimulation energy to modulate catecholamine release from chromaffin cells within the adrenal gland (104, 414, 916).

2. The implantable medical device of claim 1,
wherein the leadless body portion is configured to attach to a capsule or periadrenal connective tissue of the adrenal gland (104, 414, 916).

3. The implantable medical device of claim 2, wherein the leadless body portion is configured to attach to at least one of: periadrenal connective tissue (418), an exterior surface of the capsule of the adrenal gland (104, 414, 916), an interior surface of the capsule of the adrenal gland (104, 414, 916), and between the interior surface and the exterior surface of the capsule of the adrenal gland (104, 414, 916).

4. The implantable medical device of any of claims 1-3, wherein the leadless body portion (116) is configured to engage the adrenal gland (104, 414, 916) or periadrenal connective tissue (418) of the patient by at least one of mechanical attachment and adhesive attachment thereto.

5. The implantable medical device of claim 4, wherein the leadless body portion further comprises at least one of a barb (510), a rigid helix and at least one talon (406) for mechanical attachment thereto.

6. The implantable medical device of claim 5, wherein the leadless body portion (402) comprises a first side (414) and a second side (416), and the leadless body portion (402) comprises a plurality of talons (406) arranged on at least one of the first side (414) and the second side (416) of the leadless body portion (402).

7. The implantable medical device of claim 6, wherein the plurality of talons (406) are configured to deploy to engage the adrenal gland (414) or periadrenal connective tissue (418) in response deployment from a deployment system (502) in a deployed configuration and collapse to a delivery configuration in response to retraction within the deployment system (502).

8. The implantable medical device of claim 6, wherein at least one of the plurality of talons (406) is the electrode (404) arranged with the leadless body portion (402) and configured to deliver the stimulation energy to modulate catecholamine release from chromaffin cells within the adrenal gland (414).

9. The implantable medical device of any of claims 1-8, wherein the leadless body portion (602, 702) comprises a first side (604, 704) and a second side (606, 706), the at least one electrode is arranged on the first side (604, 704), and the leadless body portion (602, 702) further comprises a plurality of protrusions (608, 610,708) arranged on the second side (606, 706) of the leadless body portion (602, 702) and configured to attach to the adrenal gland for mechanical attachment thereto.

10. The implantable medical device of claim 9, wherein the plurality of protrusions (608, 610) are angled relative to the second side of the leadless body portion (602).

11. The implantable medical device of claim 9, wherein the plurality of protrusions (708) are perpendicular to the second side of the leadless body portion (702).

12. The implantable medical device of any of claims 1-11, wherein the leadless body portion (802) comprises a first side (804) and a second side (806), the at least one electrode is arranged on the first side (804), and the second side (806) of the leadless body portion (802) includes a roughened surface (808) configured to engage the adrenal gland for mechanical attachment thereto.

13. The implantable medical device of any of claims 1-11, wherein the leadless body portion (902) comprises a first end section (904), a second end section (908), and an intermediate section (906) between the first end section (904) and the second end section (906), and the first end section (904) and the second end section (906) having a greater flexibility than the intermediate section (906).

14. The implantable medical device of claim 13, wherein the first end section and the second end section of the leadless body portion (902) are configured to enhance engagement of the adrenal gland (916) for mechanical attachment thereto.

15. The implantable medical device of any of claims 1-14, wherein the at least one electrode comprises a patch electrode (1004) having a coiled structure with a plurality of individually insulated electrodes.

## Patentansprüche

1. Implantierbares Medizinprodukt (102, 400, 500, 600, 700, 800, 900, 1000) zum Abgeben leitungsloser Stimulationstherapie zu einer Nebenniere eines Patienten, das aufweist:
einen leitungslosen Körperabschnitt (116, 402, 504, 602, 702, 802, 902, 1002), der so konfiguriert ist, dass er einen Eingriff mit einer Nebenniere (104, 414, 916) oder periadrenalem Bindegewebe (418) eines Patienten herstellen kann; und
mindestens eine Elektrode (122, 404, 512, 914, 1004), die mit dem leitungslosen Körperabschnitt angeordnet und so konfiguriert ist, dass sie Stimulationsenergie abgibt, um Catecholaminfreisetzung aus chromaffinen Zellen in der Nebenniere (104, 414, 916) zu modulieren.

2. Implantierbares Medizinprodukt nach Anspruch 1,
wobei der leitungslose Körperabschnitt so konfiguriert ist, dass er an einer Kapsel oder periadrenalem Bindegewebe der Nebenniere (104, 414, 916) angebracht werden kann.

3. Implantierbares Medizinprodukt nach Anspruch 2, wobei der leitungslose Körperabschnitt so konfiguriert ist, dass er an periadrenalem Bindegewebe (418), einer Außenfläche der Kapsel der Nebenniere (104, 414, 916), einer Innenfläche der Kapsel der Nebenniere (104, 414, 916) und/oder zwischen der Innenfläche und der Außenfläche der Kapsel der Nebenniere (104, 414, 916) angebracht werden kann.

4. Implantierbares Medizinprodukt nach einem der Ansprüche 1 bis 3, wobei der leitungslose Körperabschnitt (116) so konfiguriert ist, dass er einen Eingriff mit der Nebenniere (104, 414, 916) oder periadrenalem Bindegewebe (418) des Patienten durch mechanische Anbringung und/oder Klebeanbringung daran herstellen kann.

5. Implantierbares Medizinprodukt nach Anspruch 4, wobei der leitungslose Körperabschnitt einen Widerhaken (510), eine starre Helix und/oder mindestens eine Kralle (406) zur mechanischen Anbringung daran aufweist.

6. Implantierbares Medizinprodukt nach Anspruch 5, wobei der leitungslose Körperabschnitt (402) eine erste Seite (414) und eine zweite Seite (416) aufweist und der leitungslose Körperabschnitt (402) mehrere Krallen (406) aufweist, die auf der ersten Seite (414) und/oder der zweiten Seite (416) des leitungslosen Körperabschnitts (402) angeordnet sind.

7. Implantierbares Medizinprodukt nach Anspruch 6, wobei die mehreren Krallen (406) so konfiguriert sind, dass sie gesetzt werden können, um einen Eingriff mit der Nebenniere (414) oder periadrenalem Bindegewebe (418) als Reaktion auf das Setzen aus einem Setzsystem (502) in einer gesetzten Konfiguration herzustellen, und in eine Abgabekonfiguration als Reaktion auf Rückzug im Setzsystem (502) kollabieren.

8. Implantierbares Medizinprodukt nach Anspruch 6, wobei mindestens eine der mehreren Krallen (406) die Elektrode (404) ist, die mit dem leitungslosen Körperabschnitt (402) angeordnet und so konfiguriert ist, dass sie die Stimulationsenergie abgibt, um Catecholaminfreisetzung aus chromaffinen Zellen in der Nebenniere (414) zu modulieren.

9. Implantierbares Medizinprodukt nach einem der Ansprüche 1 bis 8, wobei der leitungslose Körperabschnitt (602, 702) eine erste Seite (604, 704) und eine zweite Seite (606, 706) aufweist, die mindestens eine Elektrode auf der ersten Seite (604, 704) angeordnet ist und der leitungslose Körperabschnitt (602, 702) ferner mehrere Vorsprünge (608, 610, 708) aufweist, die auf der zweiten Seite (606, 706) des leitungslosen Körperabschnitts (602, 702) angeordnet und so konfiguriert sind, dass sie an der Nebenniere zur mechanischen Anbringung daran angebracht werden können.

10. Implantierbares Medizinprodukt nach Anspruch 9, wobei die mehreren Vorsprünge (608, 610) relativ zur zweiten Seite des leitungslosen Körperabschnitts (602) abgewinkelt sind.

11. Implantierbares Medizinprodukt nach Anspruch 9, wobei die mehreren Vorsprünge (708) senkrecht zur zweiten Seite des leitungslosen Körperabschnitts (702) sind.

12. Implantierbares Medizinprodukt nach einem der Ansprüche 1 bis 11, wobei der leitungslose Körperabschnitt (802) eine erste Seite (804) und eine zweite Seite (806) aufweist, die mindestens eine Elektrode auf der ersten Seite (804) angeordnet ist und die zweite Seite (806) des leitungslosen Körperabschnitts (802) eine aufgeraute Oberfläche (808) aufweist, die so konfiguriert ist, dass sie einen Eingriff mit der Nebenniere zur mechanischen Anbringung daran herstellen kann.

13. Implantierbares Medizinprodukt nach einem der Ansprüche 1 bis 11, wobei der leitungslose Körperabschnitt (902) ein erstes Endteilstück (904), ein zweites Endteilstück (908) und ein Zwischenteilstück (906) zwischen dem ersten Endteilstück (904) und dem zweiten Endteilstück (906) aufweist und wobei das erste Endteilstück (904) und das zweite Endteilstück (906) eine größere Flexibilität als das Zwischenteilstück (906) haben.

14. Implantierbares Medizinprodukt nach Anspruch 13, wobei das erste Endteilstück und das zweite Endteilstück des leitungslosen Körperabschnitts (902) so konfiguriert sind, dass sie den Eingriff der Nebenniere (916) zur mechanischen Anbringung daran verstärken kann.

15. Implantierbares Medizinprodukt nach einem der Ansprüche 1 bis 14, wobei die mindestens eine Elektrode eine Patch-Elektrode (1004) aufweist, die eine gewundene Struktur mit mehreren einzeln isolierten Elektroden hat.

## Revendications

1. Dispositif médical implantable (102, 400, 500, 600, 700, 800, 900, 1000) pour délivrer une thérapie de stimulation sans fil à une glande surrénale d'un patient comprenant :
une partie formant corps sans fil (116, 402, 504, 602, 702, 802, 902, 1002) configurée pour entrer en contact avec une glande surrénale (104, 414, 916) ou un tissu conjonctif périsurrénal (418) d'un patient; et
au moins une électrode (122, 404, 512, 914, 1004) agencée avec la partie formant corps sans fil et configurée pour délivrer une énergie de stimulation pour moduler la libération de catécholamine par les cellules chromaffines dans la glande surrénale (104, 414, 916).

2. Dispositif médical implantable selon la revendication 1,
dans lequel la partie formant corps sans fil est configurée pour se fixer à une capsule ou un tissu conjonctif périsurrénal de la glande surrénale (104, 414, 916).

3. Dispositif médical implantable selon la revendication 2, dans lequel la partie formant corps sans fil est configurée pour se fixer à au moins l'un de: le tissu conjonctif périsurrénal (418), une surface extérieure de la capsule de la glande surrénale (104, 414, 916), une surface intérieure de la capsule de la glande surrénale (104, 414, 916), et entre la surface intérieure et la surface extérieure de la capsule de la glande surrénale (104, 414, 916).

4. Dispositif médical implantable selon l'une quelconque des revendications 1-3, dans lequel la partie formant corps sans fil (116) est configurée pour entrer en contact avec la glande surrénale (104, 414, 916) ou le tissu conjonctif périsurrénal (418) du patient par au moins l'une de fixation mécanique et de fixation adhésive à celle-ci.

5. Dispositif médical implantable selon la revendication 4, dans lequel la partie formant corps sans fil comprend en outre au moins l'un d'un ardillon (510), d'une hélice rigide et d'au moins une griffe (406) pour une fixation mécanique à celle-ci.

6. Dispositif médical implantable selon la revendication 5, dans lequel la partie formant corps sans fil (402) comprend un premier côté (414) et un second côté (416), et la partie formant corps sans fil (402) comprend une pluralité de griffes (406) agencées sur au moins l'un du premier côté (414) et du second côté (416) de la partie formant corps sans fil (402).

7. Dispositif médical implantable selon la revendication 6, dans lequel la pluralité de griffes (406) sont configurées pour se déployer pour entrer en contact avec la glande surrénale (414) ou le tissu conjonctif périsurrénal (418) en réponse au déploiement d'un système de déploiement (502) dans une configuration déployée et pour s'affaisser dans une configuration de délivrance en réponse à la rétraction dans le système de déploiement (502).

8. Dispositif médical implantable selon la revendication 6, dans lequel au moins l'une de la pluralité de griffes (406) est l'électrode (404) agencée avec la partie formant corps sans fil (402) et configurée pour délivrer l'énergie de stimulation pour moduler la libération de catécholamine par les cellules chromaffines dans la glande surrénale (414).

9. Dispositif médical implantable selon l'une quelconque des revendications 1-8, dans lequel la partie formant corps sans fil (602, 702) comprend un premier côté (604, 704) et un second côté (606, 706), la au moins une électrode est agencée sur le premier côté (604, 704), et la partie formant corps sans fil (602, 702) comprend en outre une pluralité de saillies (608, 610, 708) agencées sur le second côté (606, 706) de la partie formant corps sans fil (602, 702) et configurées pour se fixer à la glande surrénale pour une fixation mécanique à celle-ci.

10. Dispositif médical implantable selon la revendication 9, dans lequel la pluralité de saillies (608, 610) sont inclinées par rapport au second côté de la partie formant corps sans fil (602).

11. Dispositif médical implantable selon la revendication 9, dans lequel la pluralité de saillies (708) sont perpendiculaires au second côté de la partie formant corps sans fil (702).

12. Dispositif médical implantable selon l'une quelconque des revendications 1-11, dans lequel la partie formant corps sans fil (802) comprend un premier côté (804) et un second côté (806), la au moins une électrode est agencée sur le premier côté (804), et le second côté (806) de la partie formant corps sans fil (802) inclut une surface rendue rugueuse (808) configurée pour entrer en contact avec la glande surrénale pour une fixation mécanique à celle-ci.

13. Dispositif médical implantable selon l'une quelconque des revendications 1-11, dans lequel la partie formant corps sans fil (902) comprend une première section d'extrémité (904), une seconde section d'extrémité (908), et une section intermédiaire (906) entre la première section d'extrémité (904) et la seconde section d'extrémité (906), et la première section d'extrémité (904) et la seconde section d'extrémité (906) ayant une plus grande flexibilité que la section intermédiaire (906).

14. Dispositif médical implantable selon la revendication 13, dans lequel la première section d'extrémité et la seconde section d'extrémité de la partie formant corps sans fil (902) sont configurées pour améliorer le contact de la glande surrénale (916) pour une fixation mécanique à celle-ci.

15. Dispositif médical implantable selon l'une quelconque des revendications 1-14, dans lequel la au moins une électrode comprend une électrode de patch (1004) ayant une structure enroulée avec une pluralité d'électrodes isolées individuellement.
